# EUROPEAN PATENT APPLICATION

(11) **EP 3 786 880 A1**
(43) Date of publication of application: **03.03.2021**
(21) Application number: 19194338.0
(22) Date of filing: 29.08.2019
(51) Int. Cl.: G06T 5/50, G06T 7/00, G16H 30/00

(54) **METHODS FOR ANALYZING AND REDUCING INTER/INTRA SITE VARIABILITY USING REDUCED REFERENCE IMAGES AND IMPROVING RADIOLOGIST DIAGNOSTIC ACCURACY AND CONSISTENCY**

(71) Applicant: Koninklijke Philips N.V., 5656 AG Eindhoven (NL)
(72) Inventor: Manikanda Krishnan, Vaidyanathan, 5656 AE Eindhoven (NL); Rao, Srinivasa, 5656 AE Eindhoven (NL)
(74) Representative: de Haan, Poul Erik

(57) **Abstract**

A method of securely accessing an image review unit, including: a triage unit configured to determine if an image of interest is normal or abnormal based upon a reference image and extract normal features from the image of interest based on normal features indicated in the reference image, wherein the reference image and the image of interest are acquired by a same medical imaging device or same doctor or same medical facility; and an image transformation unit configured to reconstruct the image of interest based upon the reference image so as to align the normal features in the image of interest with the normal features in the reference image.

## Description

### FIELD OF THE INVENTION

Various exemplary embodiments disclosed herein relate generally to methods for analyzing and reducing inter/intra site variability using reduced reference images and improving radiologist diagnostic accuracy and consistency.

### BACKGROUND OF THE INVENTION

Medical images like Chest X-rays (CXR), magnetic resonance imaging (MRI), computerized tomography (CT) scans, positron emission tomography (PET) scan, ultrasound, etc. are one of the first choices for diagnosis and are fundamental to many patient pathways revealing some unsuspected pathologic alterations. Diagnosing medical images requires careful observation and knowledge of anatomical principles, physiology and pathology and is subjected to a lot of inter-observer variability due to experience levels of the medical professional reading the medical image. Medical professionals like physicians, radiologists, and radiographer assistants reading medical images have different expertise levels due to their inability to spot exact anomalies in medical images, the anomalies appearance, and low distinguishable range of grey values in the medical image. Almost all physicians examine patients, obtain medical histories, diagnose illnesses, prescribe and treat injury or disease. A radiologist is a physician who completed medical school and received specialized training in obtaining and interpreting medical images using X-rays (radiographs, CT, fluoroscopy), radioactive substances (nuclear medicine), sound waves (ultrasound) or magnets (MRI), etc. Radiologist assistants are technicians that are experienced, registered radiographers who have obtained additional education and certification that qualifies them to serve as radiology extenders. They work under the supervision of a radiologist to provide patient care in the diagnostic imaging environment. Reviewing chest X-rays heavily depends on the experience of radiologists as there is an overlap of structures, similarity in radiographic appearances of some chest diseases, and subtlety of some chest pathologies that are not clearly distinguishable.

Neural networks have been used to analyze and classify medical images. Large scale datasets required to train a deep convolutional neural network (DCNN), like ChestX-ray14 (NIH), do not contain very accurate labels. Most of the prior deep learning work focuses on extracting and generalizing the global features. These approaches do not generalize on multi-site and multi-scanner data sources, as they will be biased towards the trained site or data sources.

### SUMMARY OF THE INVENTION

A summary of various exemplary embodiments is presented below. Some simplifications and omissions may be made in the following summary, which is intended to highlight and introduce some aspects of the various exemplary embodiments, but not to limit the scope of the invention. Detailed descriptions of an exemplary embodiment adequate to allow those of ordinary skill in the art to make and use the inventive concepts will follow in later sections.

Various embodiments relate to an image review unit, including: a triage unit configured to determine if an image of interest is normal or abnormal based upon a reference image and extract normal features from the image of interest based on normal features indicated in the reference image, wherein the reference image and the image of interest are acquired by a same medical imaging device or same doctor or same medical facility; and an image transformation unit configured to reconstruct the image of interest based upon the reference image so as to align the normal features in the image of interest with the normal features in the reference image.

Various embodiments are described, wherein the triage unit further comprises a feature extractor configured to extract a set of features from the reference image and the image of interest, and the triage unit is further configured to compute a similarity score between the features extracted from the reference image and the image of interest.

Various embodiments are described, wherein the similarity score is based upon the most relevant features extracted from the reference image and the image of interest.

Various embodiments are described, wherein the most relevant features extracted from the reference image and the image of interest are determined using a machine learning model.

Various embodiments are described, wherein determining if an image of interest is normal or abnormal includes comparing the similarity score to a threshold.

Various embodiments are described, wherein the image transformation unit further comprises a feature extractor configured to extract a set of features from the reference image and the image of interest, and the image transformation unit is further configured to compute weights for the features extracted from the reference image and the image of interest.

Various embodiments are described, wherein the image transformation unit is further configured to extract the most relevant features of the features extracted from the reference image and the image of interest and determine a set of filters associated with the most relevant features.

Various embodiments are described, wherein the weights for the features extracted from the reference image and the image of interest are computed using a machine learning model.

Various embodiments are described, wherein the image transformation unit is further configured to perform a feature alignment between the relevant features of the reference image and the image of interest.

Various embodiments are described, wherein performing the feature alignment includes solving an optimization problem to minimize the distance between the relevant features of the reference image and the image of interest such that the remaining features in the image of interest remain close to the original input image features

Various embodiments are described, wherein reconstructing the image of interest includes making some relevant features more prominent.

Various embodiments are described, wherein the reference image is selected by a user.

Various embodiments are described, wherein the reference image is selected by an algorithm based upon preselected constraints.

Further various embodiments relate to a method of processing medical images by a medical image triage and transformation system, including: determining, by a triage unit, if an image of interest is normal or abnormal based upon a reference image and extracting normal features from the image of interest based on normal features indicated in the reference image, wherein the reference image and the image of interest are acquired by a same medical imaging device or same doctor or same medical facility; and reconstructing, by an image transformation unit, the image of interest based upon the reference image so as to align the normal features in the image of interest with the normal features in the reference image.

Various embodiments are described, further including: extracting a set of features from the reference image and the image of interest; and computing a similarity score between the features extracted from the reference image and the image of interest.

Various embodiments are described, wherein the similarity score is based upon the most relevant features extracted from the reference image and the image of interest.

Various embodiments are described, wherein the most relevant features extracted from the reference image and the image of interest are determined using a machine learning model.

Various embodiments are described, wherein determining if an image of interest is normal or abnormal includes comparing the similarity score to a threshold.

Various embodiments are described, further including: extracting a set of features from the reference image and the image of interest; and computing weights for the features extracted from the reference image and the image of interest.

Various embodiments are described, further including extracting the most relevant features of the features extracted from the reference image and the image of interest and determining a set of filters associated with the most relevant features.

Various embodiments are described, wherein the weights for the features extracted from the reference image and the image of interest are computed using a machine learning model.

Various embodiments are described, further including performing a feature alignment between the relevant features of the reference image and the image of interest.

Various embodiments are described, wherein performing the feature alignment includes solving an optimization problem to minimize the distance between the relevant features of the reference image and the image of interest such that the remaining features in the image of interest remain close to the original input image features

Various embodiments are described, wherein reconstructing the image of interest includes making some relevant features more prominent.

Various embodiments are described, wherein the similarity score is based upon the most relevant features extracted from the reference image and the image of interest.

Various embodiments are described, further including: acquiring images from a plurality of scanners; performing a quality check on the acquired images; standardizing the acquired images; and storing the verified and standardized images in a storage server.

Various embodiments are described, further including creating worklists of images to be reviewed by user based upon the whether the images are normal or abnormal.

Various embodiments are described, wherein all images indicated as abnormal are place on the worklist of images to be reviewed by user.

Various embodiments are described, further including storing the similarity scores for the image of interest with the image of interest in a storage server: extracting a set of features from the reference image and the image of interest; and computing a similarity score between the features extracted from the reference image and the image of interest.

Various embodiments are described, wherein the reference image is selected by a user.

Various embodiments are described, wherein the reference image is selected by an algorithm based upon preselected constraints.

### BRIEF DESCRIPTION OF THE DRAWINGS

In order to better understand various exemplary embodiments, reference is made to the accompanying drawings, wherein:
FIG. 1 illustrates a medical image triage and transformation system;
FIG. 2 illustrates the operation of the triage unit;
FIG. 3 illustrates the operation of the image transformation unit;
FIG. 4 illustrates examples of the best performing reference image for each dataset; and
FIG. 5 illustrates an exemplary hardware diagram for implementing the medical image triage and transformation system.

To facilitate understanding, identical reference numerals have been used to designate elements having substantially the same or similar structure and/or substantially the same or similar function.

### DETAILED DESCRIPTION OF EMBODIMENTS

The description and drawings illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be for pedagogical purposes to aid the reader in understanding the principles of the invention and the concepts contributed by the inventor(s) to furthering the art and are to be construed as being without limitation to such specifically recited examples and conditions. Additionally, the term, "or," as used herein, refers to a non-exclusive or (*i.e.*, and/or), unless otherwise indicated (*e.g.*, "or else" or "or in the alternative"). Also, the various embodiments described herein are not necessarily mutually exclusive, as some embodiments can be combined with one or more other embodiments to form new embodiments.

Embodiments of a medical image triage and transformation system is described that uses DCNN models with automated triaging of chest radiographs with multi-site and multiple data sources that captures radiologist (or another medical professional) and scanner variability. The medical image triage and transformation system was validated and trained with a mix of public datasets, and then the its performance was verified on unseen multi-site and multi-source data. The medical image triage and transformation system may be implemented using a normalized Densenet-121 that maximizes like-features based on trained embedding of the source and a single reference chest X-ray, that preserves unique features of each of the image source equipment to mitigate the generalization failures on multi-site and multiple data sources.

Medical professionals face the following challenges with the complexity of interpretation of CXRs. While chest X-rays are used as an example herein, other types of X-rays and medical images may be used as well. Reviewing chest X-rays heavily depends on the experience of radiologists. Reasons for this challenge include but are not limited to anatomical noise caused by superimposition of thoracic structures, similarity in radiographic appearances of some chest diseases, subtlety of some chest pathologies that are clearly indistinguishable, site specific practices, and scanner variabilities. The radiologist faces time pressures. Each chest X-ray takes a trained radiologist several minutes to review and write the report. Many radiologists have to work over-time, increasing the misdiagnosis due to exhaustion. It is reported that about 20% to 50% of lung nodules are missed or misdiagnosed on chest X-rays, while most of these missed or misdiagnosed lung nodules may be detected retrospectively or by a second reviewer.

Another challenge it the disagreement between radiologists in reading chest X-rays. In a comparative study of casualty plain radiographs, significant disagreement varied between 5 to 9%. The presence of an abnormality rather than its significance was more likely to give rise to observer variation. Such errors may be defined as perceptual or cognitive. Perceptual errors occur when image features though recorded were not appreciated. Reporting errors may also be classified as either false positive or false negative. A false positive study is an interpretation of a feature as pathological which is in fact normal or a normal variant. A false negative or under-reading may be a finding that is present but is missed. False negative errors are five times more common than false positive errors. Similarly, perceptual errors are four times more frequent than cognitive errors.

Scanning technique and scanner equipment issues can cause variations in the images captured. The penetration of a CXR is affected by the duration of exposure and the power of the beam. A poorly penetrated CXR looks light, and soft tissue structures are hard to see, especially those behind the heart. An over penetrated CXR looks dark and lung markings are hard to see. When a CXR has good penetration, the lower thoracic vertebral bodies may be seen through the heart.

Automating the analysis of CXR images would be very beneficial, but various challenges exist in automating CXR image analysis. For example, inaccuracy in labelling for automation due to radiologist experience level. In order to train a medical image triage and transformation system that analyzes CXR images, the creation of high quality dataset having a size that is orders of magnitude larger than current datasets is needed to drive the field forward. On large data sets, like ChestX-ray14 (NIH), the labelling of images as normal or abnormal are not very accurate. One of reasons could be because the labelling process is automated by reading of radiology reports by a natural language processing (NLP) based algorithm. In doing so, an additional layer of error is introduced into the ground truth.

Another challenge in automating CXR analysis is the generalization of deep learning models in multi-site environment: Most of the deep learning work to date has focused on extracting and generalizing the global features like boundary, shape, edge properties and does not focus on normal and abnormal classification for triaging purposes. That is, if the automates system can determine if an image is normal or abnormal, then the workload of the radiologist may be reduced. One proposed system used a combination of manually extracted features and learned features in a convolutional neural network (CNN) to achieve lung nodule classification with high specificity. Another system uses a three-phase approach, where the focus is on classification of abnormalities based on NIH dataset alone. This system focuses on a region of interest to extract the local features like texture patterns and finer structures, to further improve abnormality classification assuming that the region of interest is identified by the global network. Yet another system uses a VGG-16 network for binary classification to determine normal versus abnormal CXR images.

Although further analysis of a region of interest generated by a deep learning model could help to a certain extent in classification, it does not generalize well, due to the labelling inconsistencies and multi-site data which has multi-observer and multi-scanner variabilities.

The medical image triage and transformation system described herein differs from the previous methods. Given that the medical image triage and transformation system is not aiming to classify images to specific categories of disease, but instead focuses on generalizing the binary classification performance by training the network to compare discriminative features from multiple sites and imaging devices. The medical image triage and transformation system may be implemented using any model like a DCNN maximizing like-features based on trained embedding of the source and a single reference CXR. This approach preserves the unique features of each of the source devices mitigating the generalization failures on multi-site and multiple data sources. Machine learning models have difficulty adapting to the following: inter scanner variability of acquired radiographs; intra site and inter site preferences for normalcy; and normalizing the viewed images across scanners. The medical image triage and transformation system described herein overcomes these problems. This is done by using a reference image that will take an input image of interest and reconstruct it resulting in images that are different for each reference image. This medical image triage and transformation system automatically learns to reweigh the disentangled aspects of the image and choose only those that are in accordance with the reference.

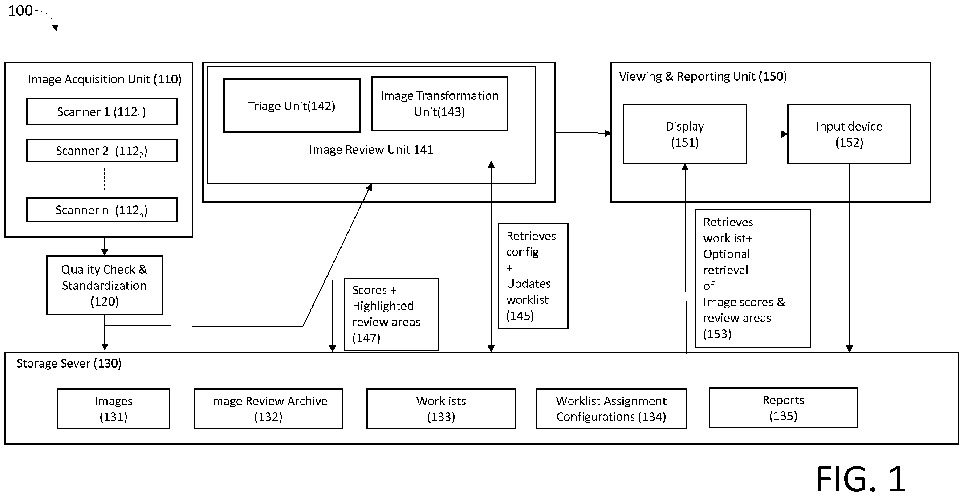

FIG. 1 illustrates a medical image triage and transformation system. The medical image triage and transformation system 100 includes an image acquisition unit 110, an image review unit 141, a view and reporting unit 150, and a storage server 130. The medical image triage and transformation system 100 studies and optimizes worklist assignment to aid and improve diagnosis. FIG. 1 illustrates how the medical image triage and transformation system fits into the workflow in a hospital.

The image acquisition unit 110 is a hardware device that interfaces with each scanner 112 or medical imaging device and receives new images from each of the scanners. The scanners 112 may be any type of medical imaging device including for example, X-ray machines, magnetic resonance imaging (MRI) machines, ultrasound machines, etc. The image acquisition unit 110 may be connected to a network that is connected to each of the scanners 112.

The storage server 130 may store medical images 131 received from the image acquisition unit 110. The storage server may be any type of storage server that includes processing and data storage devices. The storage server 130 may also include an image review archive 132 that collects images that need to be reviewed by a radiologist or technician. The storage server 130 also includes work lists that take the images in the image review archive 132 and forms list of images to be reviewed by radiologists and technicians. This list of images may be sorted according to various factors including but not limited to time and scores provided by the image review unit 141. This list of images may also be subdivided into multiple lists of various priorities including but not limited to urgent, mild and observational. Such worklists 133 may spread the workload of images to be reviewed among the various radiologist and technicians available. Also, such worklist may collect certain types of images that need to be reviewed by specific radiologists or technician. This may be based upon whether the image is classified as normal or abnormal by a triage unit 142 or further by the type of image or the location of the body being imaged. The triage unit 142 is a system that determines whether a image to be reviewed is normal or abnormal. Thus, the triage unit 142 helps to triage the images to be reviewed based upon the classification as normal or abnormal. With such a classification the images to be reviewed may be prioritized so that the images needed the most care in review get the attention needed. Hence, the images are "triaged" by the triage unit 142. This process will be further described below.

The storage server 130 may also include worklist assignment configurations 134. These worklist configurations 134 help to determine how to assign images to reviewed in the image review archive 132 to specific worklists 133. These worklist assignment configuration 134 may specify parameters and rule to make the worklists 133. The storage server 130 may also store reports 135 related to each image. When images are evaluated the radiologist writes a report regarding the evaluation of the image which is then stored in the storage server. The reports 135 are then available to other physicians or medical personnel for use in treating and evaluating patients.

The viewing and reporting unit 150 is a hardware device including a display 151 and an input unit 152. The display 151 is any display capable of displaying the images 131 stored in the storage server 130. The input device 152 may include a keyboard or other text entry device that allows for the radiologist or technician to write a report evaluating each image. Also, the viewing and reporting unit 150 may also include an input device 152 that allows for the annotation of the images evaluated, such as pointing out specific features of the image that may indicate disease or anomalies. The input device 152 could also be implemented on the display 151 by a touch sensitive surface.

The image review unit 141 helps facilitate the review of images by a radiologist or technician. Going forward when review of a medical image by a radiologist is mentioned, such review may also be carried out by a technician in certain circumstances. The image review unit 141 may include a triage unit 142 and image transformation unit 143 each of which will be explained in greater detail below. The image review unit 141 is a hardware device that includes processing, data storage, communication interfaces, and the image review unit 141 may use software instructions to carry out its functions.

The operation of the medical image triage and transformation system will now be described. The digital images captured from the various scanners 112 are checked for quality and standardized 120. The image is then stored in the storage server 131, and the images are also passed to the image review unit 141. The image review unit 141 performs two operations. The new images are triaged by the triage unit 142 that uses a normalized binary classifier and a reference image to determine if an image is normal or abnormal. The reference image may be selected by a user or by an algorithm based upon preselected constraints. Further, the images are transformed by the image transformation unit 143 by normalizing the image based upon a reference image. These outputs are then stored 143 in the image review archive 132 along with the scores and review areas generated by image review unit 141 for later use by a user of the system. If requested by a user, the viewing and reporting unit 150 retrieves the images 145 from the image review archive 132 and displays it to the user. The specific images assigned to a user to review may be based upon the worklists 133 that are defined by the worklist assignment configurations 134. The user may then create reports and diagnose the case associated with each image which is then stored into the reports 135 section of the storage server 130.

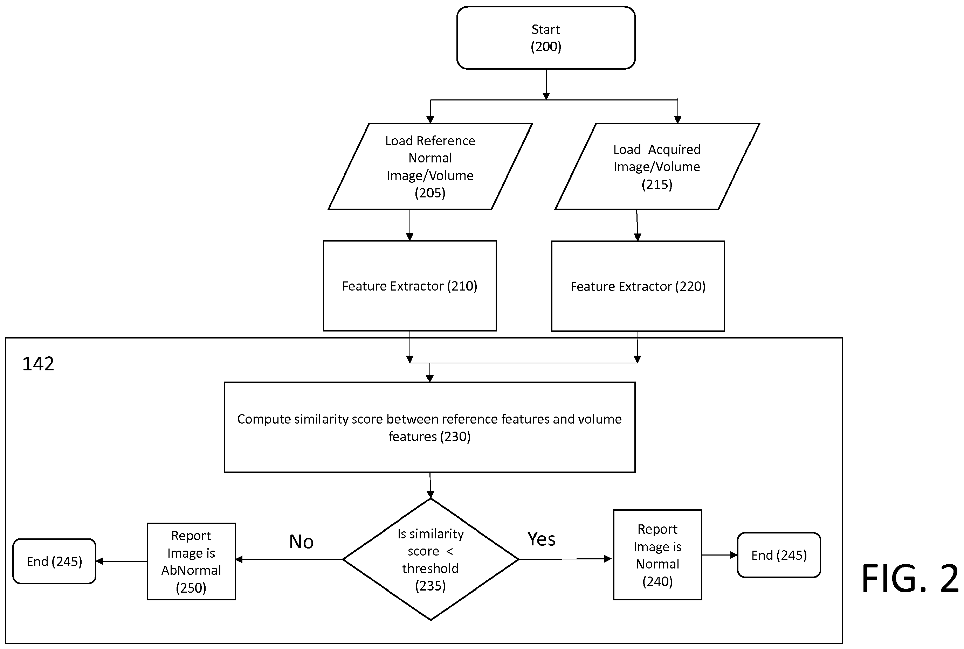

FIG. 2 illustrates the operation of the triage unit 142. The triage unit 142 loads reference image(s) 205 and extracts features *fᵣ* from the reference images 210. The triage unit 142 also loads a multi-site medical volume 215 from the medical images and extract features *fᵢ* 220 from the images of interest. The feature extraction can be accomplished by algorithms including but not limited to a trained machine learning model such as a convolutional neural network (CNN), statistical models extracting local descriptors such as entropy, mean, variance etc. The triage unit 142 then computes a similarity score between the reference image features and loaded multisite dataset volume and validates the image with reduced-reference image quality assessment metrics 230. The function of the similarity score is to quantify the closeness between the reference image and image of interest according to a distance function, some examples of distance functions would be Euclidean distance, cosine distance etc. Next, the output is compared to a threshold which is specified based on operational requirements such as but not limited to high sensitivity and high specificity, to make a binary classification decision 235. The binary classification decision is made purely based on similar features out-weighing the dis-similar features in the images. Further, the comparison may be done between a reference image from the same scanner that was used to take the image being triaged. This leads to a reduction in the effects of classifying images due to variations between scanners. If the similarity score is below the threshold, then the image is reported as being abnormal 250 and the process ends 245. If the similarity score is above the threshold, then the image is reported as being normal 240 and the process ends 245. This triaging of the images allows for abnormal images to be identified and either be given priority or to be first given to the radiologist. Normal images may be given a lower priority or may be first read by a technician.

When using the triage unit 142 false negatives should ideally be zero. The triage unit 142 can never be 100% accurate. However, even if a few images are mislabeled, there is little to no impact on the diagnosis itself, because low scoring images are still assessed manually. By identifying additional review areas in an image (as described below), it may take a junior radiologist additional time to look at and assess these regions. However, this comes with an added benefit of reduced likelihood of false negatives.

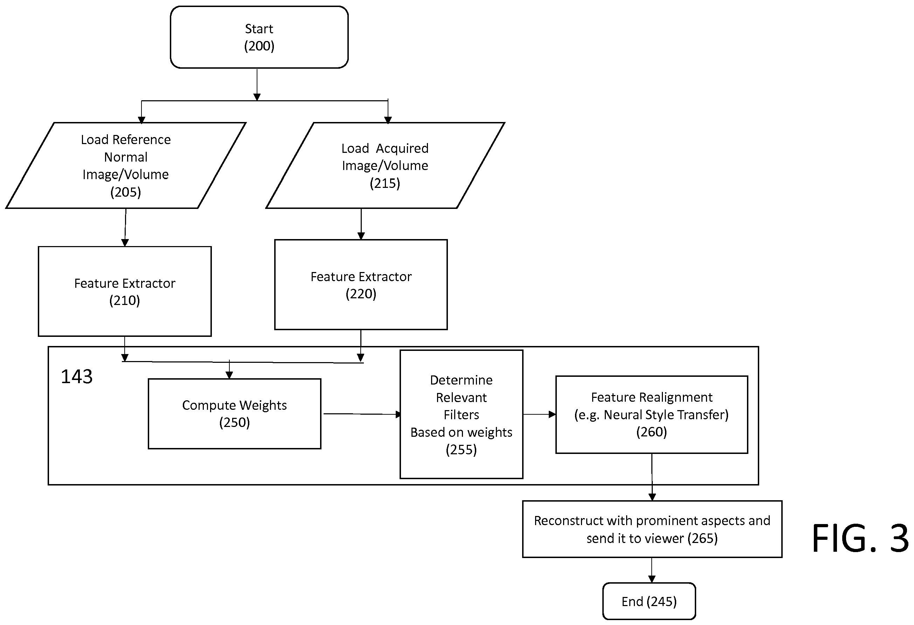

FIG. 3 illustrates the operation of the image transformation unit 142. The image transformation unit 142 takes in the same extracted features from the feature extractors 210 and 220 as described above with respect to the triage unit 142. The image transformation unit 142 may then compute weights for the features of the image of interest *fᵢ*. This may be done by concatenating the reference image features *fᵣ* and the image of interest features *fᵢ*, and then passing the combined features into a neural network that computes the weights for *fᵢ* to rank the features to determine which features are relevant and which are non-relevant. Next, the image transformation unit 142 determines the relevant features based upon the weights and determines a set of filters 255. Then the image transformation unit 142 performs a feature realignment 260. The feature realignment may be done by solving an optimization problem to minimize the distance (using any chosen distance function) between the relevant features *fᵣₖ* and *fᵢₖ* such that the remaining features in the image of interest remain close to the original input image features according to a similarity or distance function such as Euclidean distance or cosine distance. Such closeness be defined by a closeness parameter to put an upper limit on the closeness. Finally, the image of interest may be reconstructed 265 based upon the realigned features and the realigned images sent to the viewing and reporting unit 150. This reduces variability in similar features across images while maintaining and enhancing the prominence of the unique structures of the original image that will assist the user in evaluating the image of interest. Because the reconstructed images have made certain features more prominent, they are more likely to be identified and promotes consensus among the radiologist reading the image and assists the less experienced among thee radiologist to perform better diagnosis, which helps to overcome the problems of variability in evaluating images by different radiologists. The image may be reconstructed using neural style transfer.

Because reference images from a specific scanner are used above, the medical image triage and transformation system 100 may address the problem of inter scanner variability by comparing the similarity of image of interest with a reference image acquired from the same scanner. Any similarity score can be used, for example cosine distance or reduced-reference image quality assessment metrics.

Further, inter site variations as well as doctor variations may be addressed based upon choosing the appropriate reference images based upon site or doctor.

Now results of an example for implementing medical image triage and transformation system showing the benefits of such a system will be described. First the data used will be described. The training/dev sets were labelled by NLP labelers. The training data used are as follows:
Chexpert Dataset [D1] having around 200K scans; and
a subset of ChestX-ray14 (NIH)[D2] dataset of around 50K samples.

The evaluation sets were all labelled by radiologists. The following six evaluation sets were used:
Chexpert Valid[E1] consisting of 234 scans;
Montgomery Dataset[E2] consisting of 138 scans;
Shenzhen Dataset[E3] consisting of 662 scans;
Private Hospital Dataset 1[E4] consisting of 3587 scans;
Private Hospital Dataset 2[E5] consisting of 200 scans; and
A 800 scan subset of ChestX-ray14(NIH) [E6] that were not part of the training set which
   were relabeled by radiologists.

The data was pre-processed. Each image was standardized between 0 and 1. The images were clipped base upon its 3 percentile and 98 percentile values before standardization in order to account for exposure issues, and the images were resized to 320x320. Any single channel input is converted into three channel inputs by passing the original input, a negated input, and vessels enhanced input as three channels. Each channel is equalized separately and normalized to 0 mean and 1 standard deviation

Various experiments in the literature have demonstrated the superior performance of the DenseNet-121 architecture as compared to other existing architectures. Thus, the evaluation uses the standard DenseNet-121 architecture as the baseline as described by: Rajpurkar, P., Irvin, J., Zhu, K., Yang, B., Mehta, H., Duan, T., Ding, D., Bagul, A., Langlotz, C., Shpanskaya, K., et al.: Chexnet: Radiologist-level pneumonia detection on chest x-rays with deep learning. arXiv preprint arXiv:1711.05225 (2017); and Wang, X., Peng, Y., Lu, L., Lu, Z., Bagheri, M., Summers, R.M.: Chestx-ray8: Hospital-scale chest x-ray database and benchmarks on weakly-supervised classification and localization of common thorax diseases. In: 2017 IEEE Conference on Computer Vision and Pattern Recognition (CVPR), IEEE (2017) 3462-3471.

The following changes are made to the baseline architecture. All batch normalization layers are replaced with group normalization layers with 32 channels forming a group. This was done to mitigate any data source specific statistics that might be computed based on training batches. Secondly the abnormality predictions were considered as the conditional probability of the abnormal class given the image is predicted as abnormal. This induces a two-level hierarchy with the first level being normal/abnormal and the second level consisting of the abnormalities. The third change involves using squeeze excitation layers; this acts like a channel attention scheme and can dynamically give weight to useful features during runtime.

The usage of a reference image by the medical image triage and transformation system 100 may be readily modelled using a Siamese architecture which is a type of neural network that learns to compare two inputs based on a distance metric such that inputs that are closer in some semantics get a lower distance in comparison to two inputs that are further apart according to the same semantics, the semantics that need to be captured is fed to the network implicitly during the training processes. The network was initialized with the weights learned by the modified architecture used in experiment 3. The network was trained with samples from D1 and D2 with normal anchors, i.e., to minimize the distance between normal samples and maximize the distance between normal and abnormal samples. Note that the network did not try to minimize the distance between abnormal samples as they are from a variety of classes and doing so might lead to poorer learning.

The following Table 1 presents results for various experiments using various models.

**Table1: Results for AUC and TPR of Normal/Abnormal Class**

| | **Model** | **Training set** | **Chexpert Validation** AUC/TPR @TNR=97% | **Montgomery** AUC/TPR @TNR=97% | **Shenzhen** AUC/TPR @TNR=97% | **Private Dataset 1** AUC/TPR @TNR=97% | **Private Dataset 2** AUC/TPR @TNR=97% | **NIH** AUC/TPR @TNR=97% |
|---|---|---|---|---|---|---|---|---|
| Experiment 1 | **DenseNet-121 (Chexpert version )** | Chexpert | 0.90/0.24 | 0.7/0.20 | 0.65/0.01 | 0.85/0.06 | 0.73/0.08 | 0.88/0.2 |
| Experiment 2 | **DenseNet-121 Modified** | Chexpert | 0.90 | 0.82 | 0.68 | 0.84 | 0.76 | 0.87 |
| Experiment 3 | **DenseNet-121 Modified** | Chexpert + NIH | 0.90/0.34 | 0.88/0.08 | 0.74/0.05 | 0.93/0.38 | 0.85/0.10 | 0.92/0.42 |
| Experiment 4 | **Normalized PenseNet. 121 Modified** | Chexpert + NIH | 0.9/0.39 | 0.91/0.41 | 0.77/0.16 | 0.93/0.51 | 0.86/0.27 | 0.92/0.44 |

The results are presented to show unique experiments including, a combination of model and training set columns. For example, Model = "DenseNet-121(Chexpert Version)" and Training Set = "Chexpert" is Experiment 1, that is validated on all different validation datasets (from column 3 to column 8) which is the baseline model. Experiments 2 and 3 show variations of the baseline model. Experiments 4 show the results for the medical image triage and transformation system 100 described herein.

The metrics used to measure the performance of the model are AUC and TPR@TNR=97%. These 2 metrics are shown in the above table as a/b where:
a denotes the receiver operating characteristic (AUC) score which qualitatively signifies whether a threshold can be found that can completely segregate the two classes; and
b denotes the TPR@TNR=97% is useful mainly in the triage settings. TNR stands for the true negative rate or the number of abnormal samples that have been marked as abnormal by the algorithm. TPR stands for true positive rate or the number of normal samples that have been marked as normal, these samples are what would be removed from his workflow. Ideally this should be 1.0 which implies that it is possible to remove all normal images from the radiologist workflow by misclassifying only 3% of the total abnormal samples in the workflow. If this metric is poor, it basically means that the model is unable to remove a significant fraction of the normal samples in the radiologist workflow.

The following conclusions can be drawn from the above Table 1:
1. The results in Experiment 1, the baseline model shows degradation from 0.90/0.24 on Chexpert validation data to 0.65/0.01 on Shenzhen data.
   a. Chexpert validation data comes from the same site as the training data used in Experiment 1 for training the model
   b. Shenzhen data comes from different site and geography.
   We can infer that Experiment 1 baseline model performs well on the validation data from same site but is poor when run on other sites like the Shenzhen data.
2. Experiment 2 is similar to Experiment 1 but tries to solve the problem by improving the model variance, thereby generating a new model.
   It can be inferred that Experiment 2 model, which is a better model compared to Experiment 1, still suffers with the degradation.
3. Experiment 3 is performed by keeping the Experiment 2 model and changing the training strategy, that is (Chexpert + NIH). NIH contains data from multiple sites.
   It can be inferred that AUC metric improves dramatically across the datasets. This is not a robust and sufficient metric for triaging. Hence TPR@TNR=97% metric is also used. It can be seen that there is still a large degradation in this metric.
4. Experiment 4 solves the degradation in the second metric by using the medical image triage and transformation system 100 described herein.
   It can be inferred that AUC metric is similar to Experiment 3 results, but there is a significant improvement in the TPR@TNR=97% metric across the datasets.

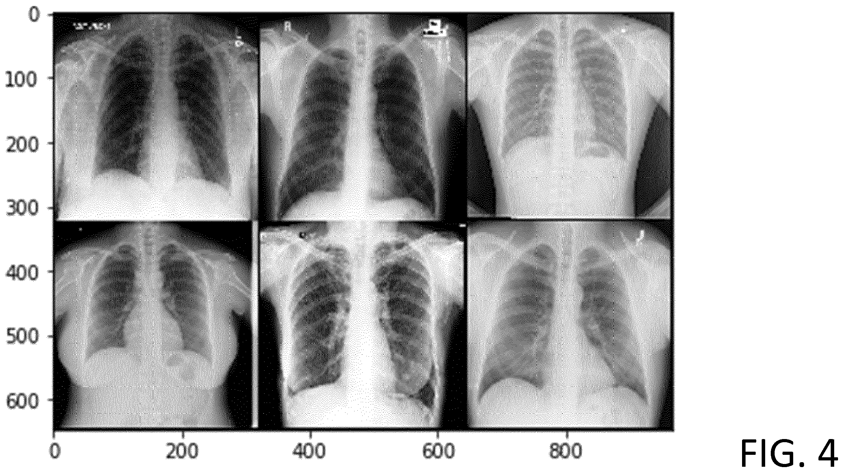

FIG. 4 illustrates the best performing references image for each dataset. It can be seen that each of the references are barely within normal limits. From the model's perspective this might imply that hard normal images have a higher discriminating power compared to good normal images.

The medical image triage and transformation system 100 described herein may be used in various settings and provide various benefits and solutions to technical problems. It may be used to implement a normalization strategy in the classification, segmentation, detection tasks on all medical image volumes. This allows for normal and abnormal images to be identified, which then can be used to manage the workload of radiologists. This can reduce the amount of time that the radiologist has to spend with each image which increases the productivity of the radiologist. The normalization and transformation of images as described herein help to make the images look more uniform over different scanners and sites for an radiologist assessing the images. This improved uniformity allows for better results in reading medical images and overcomes the problem of variations in images from different scanners as well as adapting to different radiologists variations in reading images. The proposed worklist assignment based on predicted abnormality scores can be used to make better use of the time of highly skilled radiologists. Again this benefits in increase productivity of the radiologists. Also, the additional provision for identifying a review region by the image transformation unit during radiologist review of the medical is useful for understanding what caused the model to give the particular score. By doing so, the radiologist can easily assess and rule out any mistakes made by the medical image triage and transformation system. Further, the triage unit can provide abnormality scores and review regions that can be viewed as a second opinion for junior radiologists. This improves the overall quality of the evaluation of medical images across a set of radiologists with varying skill and experience levels. This system also improves the overall evaluation accuracy of the medical images.

A further technological advantage is the triaging done by the medical image triage and transformation system. The the medical image triage and transformation system uses a clustering approach (by moving one class of the classification- Normal images, more nearer to the NORMAL reference image of the hospital site) in classification. This step is important to counter and capture the differences and variations present in the images generated by various sites and scanners. This approach will ensure that the classifier adapts to the variations present in the images generated by various sites and scanners with various intensity levels, as it uses clustering as a sub step of classification in optimizing the triage process. Optimization is designed to implicitly learn features that can compares the reference image and input image and outputs a similarity score, as a sub-step to help the main classification using a deep learning approach. This helps the triaging deep learning model to generalize better and work with almost same accuracy, across sites, reducing the effect of variations across sites and builds a better ready to deployable model that works across sites.

A technical advantage/purpose of generating an enhanced input image include the following. Scanner/PACS/Workstation viewing image quality is improved. Also, a few artefacts may be corrected. The medical image triage and transformation system generates an image and helps standardize the image across various scanners so that images from different scanners visually look similar to the reference without affecting the finer features. This leans to a similar diagnosis decision by different experienced radiologists, as the corrected images show the structures in the image uniformly, across sites and scanners. It also makes the review of medical images by radiologist easier. The medical image triage and transformation system reduces disagreement between various experience level radiologists, and helps them to make similar decisions, as the parenchyma is more clearly visible. Also, analyzing and correcting inter/site/intra-site and inter/intra-scanner variability in medical images shall reduce inter observer variability, improving radiologist diagnostic accuracy and consistency. This approach works with the same trained deep learning model, without retraining on multiple data sources and multiple sites, with a few images of tuning. As the generated image is more uniform, the traditional image processing software's work performs better, as they are mostly based on intensities, patterns and thresholding.

The examples discussed above relate to chest X-Rays, but the medical image triage and transformation system described herein may be extended to other modalities.

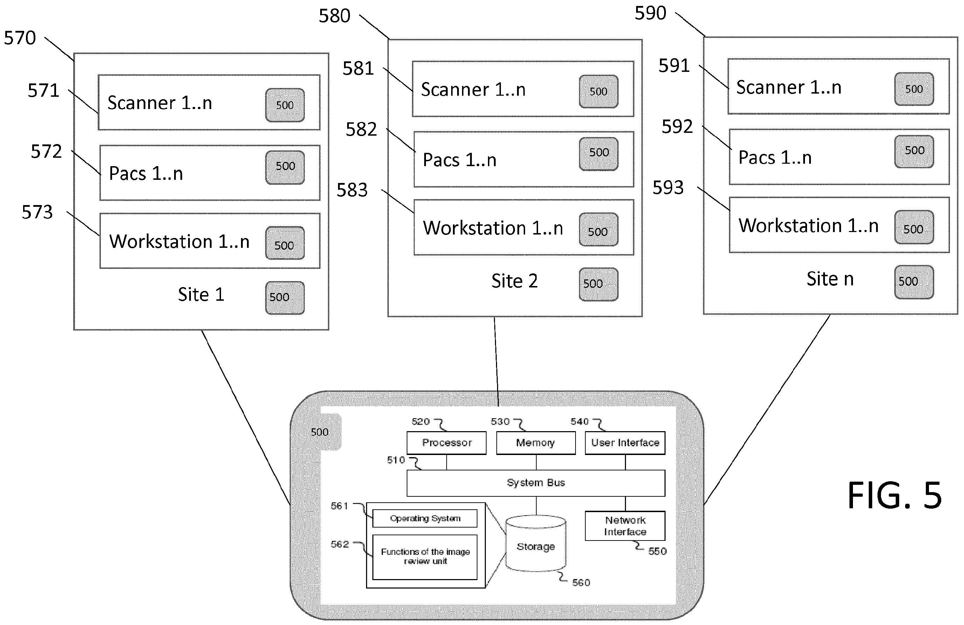

FIG. 5 illustrates an exemplary hardware diagram for implementing the medical image triage and transformation system. Further, as shown the device 500 may be used to implement the image review unit 141, the image acquisition unit 110, the viewing and reporting unit 150, or the storage server 130. As shown, the device 500 includes a processor 520, memory 530, user interface 540, network interface 550, and storage 560 interconnected via one or more system buses 510. It will be understood that FIG. 5 constitutes, in some respects, an abstraction and that the actual organization of the components of the device 500 may be more complex than illustrated.

Further, the device 500 may be implemented in various parts of the medical image triage and transformation system as shown in FIG. 5. The medical image triage and transformation system may include three sites 570, 580, and 590. Note that more sites are also anticipated. Each site may include a set of scanners 571, 581, 591 that each may include the device 500. Further, each site may include a set of Picture Archive and Communication System (PACS) 572, 582, 592 that each may include the device 500. Finally, each site may include a set of workstations 573, 583, 593 that each may include the device 500.

The processor 520 maybe any hardware device capable of executing instructions stored in memory 530 or storage 560 or otherwise processing data. As such, the processor may include a microprocessor, field programmable gate array (FPGA), application-specific integrated circuit (ASIC), or other similar devices.

The memory 530 may include various memories such as, for example L1, L2, or L3 cache or system memory. As such, the memory 530 may include static random-access memory (SRAM), dynamic RAM (DRAM), flash memory, read only memory (ROM), or other similar memory devices.

The user interface 540 may include one or more devices for enabling communication with a user. For example, the user interface 540 may include a display, a touch interface, a mouse, and/or a keyboard for receiving user commands. In some embodiments, the user interface 540 may include a command line interface or graphical user interface that may be presented to a remote terminal via the network interface 550. The user interface 540 may implement the viewing and reporting unit 150.

The network interface 550 may include one or more devices for enabling communication with other hardware devices. For example, the network interface 550 may include a network interface card (NIC) configured to communicate according to the Ethernet protocol or other communications protocols, including wireless protocols. Additionally, the network interface 550 may implement a TCP/IP stack for communication according to the TCP/IP protocols. Various alternative or additional hardware or configurations for the network interface 550 will be apparent.

The storage 560 may include one or more machine-readable storage media such as read-only memory (ROM), random-access memory (RAM), magnetic disk storage media, optical storage media, flash-memory devices, or similar storage media. In various embodiments, the storage 560 may store instructions for execution by the processor 520 or data upon with the processor 520 may operate. For example, the storage 560 may store a base operating system 561 for controlling various basic operations of the hardware 500. The storage 561 may store instructions for carrying out the functions of the of the image review unit 141, the triage unit 142, or the image transformation unit 143. Also, the storage 562 may also store the instructions for carrying out the functions of the image acquisition unit 110, the storage server 130, or the viewing and reporting unit 150.

It will be apparent that various information described as stored in the storage 560 may be additionally or alternatively stored in the memory 530. In this respect, the memory 530 may also be considered to constitute a "storage device" and the storage 560 may be considered a "memory." Various other arrangements will be apparent. Further, the memory 530 and storage 560 may both be considered to be "non-transitory machine-readable media." As used herein, the term "non-transitory" will be understood to exclude transitory signals but to include all forms of storage, including both volatile and non-volatile memories.

While the host device 500 is shown as including one of each described component, the various components may be duplicated in various embodiments. For example, the processor 520 may include multiple microprocessors that are configured to independently execute the methods described herein or are configured to perform steps or subroutines of the methods described herein such that the multiple processors cooperate to achieve the functionality described herein. Further, where the device 500 is implemented in a cloud computing system, the various hardware components may belong to separate physical systems. For example, the processor 520 may include a first processor in a first server and a second processor in a second server.

Any combination of specific software running on a processor to implement the embodiments of the invention, constitute a specific dedicated machine.

As used herein, the term "non-transitory machine-readable storage medium" will be understood to exclude a transitory propagation signal but to include all forms of volatile and non-volatile memory.

Although the various exemplary embodiments have been described in detail with particular reference to certain exemplary aspects thereof, it should be understood that the invention is capable of other embodiments and its details are capable of modifications in various obvious respects. As is readily apparent to those skilled in the art, variations and modifications can be affected while remaining within the spirit and scope of the invention. Accordingly, the foregoing disclosure, description, and figures are for illustrative purposes only and do not in any way limit the invention, which is defined only by the claims.

## Claims

1. An image review unit, comprising:
a triage unit configured to determine if an image of interest is normal or abnormal based upon a reference image and extract normal features from the image of interest based on normal features indicated in the reference image, wherein the reference image and the image of interest are acquired by a same medical imaging device or same doctor or same medical facility; and
an image transformation unit configured to reconstruct the image of interest based upon the reference image so as to align the normal features in the image of interest with the normal features in the reference image.

2. The image review unit of claim 1, wherein
the triage unit further comprises a feature extractor configured to extract a set of features from the reference image and the image of interest, and
the triage unit is further configured to compute a similarity score between the features extracted from the reference image and the image of interest.

3. The image review unit of claim 2, wherein
the similarity score is based upon the most relevant features extracted from the reference image and the image of interest,
the most relevant features extracted from the reference image and the image of interest are determined using a machine learning model, and
determining if an image of interest is normal or abnormal includes comparing the similarity score to a threshold

4. The image review unit of claim 1, wherein
the image transformation unit further comprises a feature extractor configured to extract a set of features from the reference image and the image of interest, and
the image transformation unit is further configured to compute weights for the features extracted from the reference image and the image of interest.

5. The image review unit of claim 6, wherein the image transformation unit is further configured to extract the most relevant features of the features extracted from the reference image and the image of interest and determine a set of filters associated with the most relevant features.

6. The image review unit of claim 7, wherein the image transformation unit is further configured to perform a feature alignment between the relevant features of the reference image and the image of interest and make some relevant features more prominent.

7. The image review unit of claim 6, wherein performing the feature alignment includes solving an optimization problem to minimize the distance between the relevant features of the reference image and the image of interest such that the remaining features in the image of interest remain close to the original input image features

8. A method of processing medical images by a medical image triage and transformation system, comprising:
determining, by a triage unit, if an image of interest is normal or abnormal based upon a reference image and extracting normal features from the image of interest based on normal features indicated in the reference image, wherein the reference image and the image of interest are acquired by a same medical imaging device or same doctor or same medical facility; and
reconstructing, by an image transformation unit, the image of interest based upon the reference image so as to align the normal features in the image of interest with the normal features in the reference image.

9. The method of claim 8, further comprising:
extracting a set of features from the reference image and the image of interest; and
computing a similarity score between the features extracted from the reference image and the image of interest.

10. The method of claim 9, wherein
the similarity score is based upon the most relevant features extracted from the reference image and the image of interest,
the most relevant features extracted from the reference image and the image of interest are determined using a machine learning model, and
determining if an image of interest is normal or abnormal includes comparing the similarity score to a threshold.

11. The method of claim 8, further comprising:
extracting a set of features from the reference image and the image of interest; and
computing weights for the features extracted from the reference image and the image of interest.

12. The method of claim 11, further comprising extracting the most relevant features of the features extracted from the reference image and the image of interest and determining a set of filters associated with the most relevant features.

13. The method of claim 12, wherein the weights for the features extracted from the reference image and the image of interest are computed using a machine learning model.

14. The method of claim 13, further comprising performing a feature alignment between the relevant features of the reference image and the image of interest and making some relevant features more prominent.

15. The method of claim 14, wherein performing the feature alignment includes solving an optimization problem to minimize the distance between the relevant features of the reference image and the image of interest such that the remaining features in the image of interest remain close to the original input image features
